# EUROPEAN PATENT APPLICATION

(11) **EP 4 445 833 A1**
(43) Date of publication of application: **16.10.2024**
(21) Application number: 22904128.0
(22) Date of filing: 01.12.2022
(51) Int. Cl.: A61B 5/02

(54) **PULSE WAVE MEASUREMENT DEVICE**

(30) Priority: 09.12.2021 JP 2021199963
(71) Applicant: Minebea Mitsumi Inc., Kitasaku-gun, Nagano 3890293 (JP)
(72) Inventor: NAGAI, Takuya, Kitasaku-gun, Nagano 389-0293 (JP)
(74) Representative: Zabel, Julia Elisabeth
(86) International application number: PCT/JP2022/044357
(87) International publication number: WO 2023/106197

(57) **Abstract**

This pulse wave measurement device can be worn by a subject and has: a pulse wave sensor having a strain gauge; a sensor disposition portion having the pulse wave sensor disposed thereon; a first curved portion joined at one end to the sensor disposition portion; a second curved portion joined at one end to the sensor disposition portion; and a band connected to the other end of the first curved portion and to the other end of the second curved portion, wherein the first curved portion and the second curved portion, in a planar view, are joined to opposite sides so as to sandwich the sensor disposition portion therebetween, and the curvatures of the first curved portion and the second curved portion are different.

## Description

### Technical Field

The present disclosure relates to a pulse wave measuring device.

### Background

A pulse wave sensor, which detects a pulse wave generated when a heart pumps blood, is known. As an example, a pulse wave sensor is provided, which has a pressure receiving plate as a strain body supported to be flexibly deflected by an external force, and a piezoelectric conversion means for converting the deflection of the pressure receiving plate into an electric signal. In this pulse wave sensor, a flexible region of the pressure receiving plate is formed into a dome-like shape with a convex curved surface outward, and a pressure detecting element, as a piezoelectric conversion means, is provided on an inner surface of the top of the pressure receiving plate (See, e.g., Patent Document 1).

### Related-art Documents

### Patent Document

Patent Document 1: Japanese Patent Publication No. 2002-78689

### Summary

### Problem to be Solved by the Invention

It is necessary for a pulse wave sensor to detect minute signals. Therefore, in a pulse wave measuring device using a pulse wave sensor, the pulse wave sensor needs to be stably arranged near a radial artery of a subject to be measured, so as to improve measurement accuracy.

In view of the point described above, an object of the present disclosure is to provide a pulse wave measuring device in which a pulse wave sensor can be stably arranged near a radial artery of a subject to be measured.

### Means to Solve the Problem

A pulse wave measuring device according to an embodiment of the present disclosure is a pulse wave measuring device attachable to a subject to be measured, and comprises: a pulse wave sensor having a strain gauge; a sensor placement member in which the pulse wave sensor is arranged; a first curved member having one end connected to the sensor placement member; a second curved member having one end connected to the sensor placement member; and a band member connected to another end of the first curved member and another end of the second curved member, wherein the first curved member and the second curved member are connected to opposite sides across the sensor placement member in a plan view, and wherein a curvature of the first curved member is different from a curvature of the second curved member.

### Effects of the Invention

According to the disclosed technique, a pulse wave measuring device, in which a pulse wave sensor can be stably arranged near a radial artery of a subject to be measured, can be provided.

### Brief Description of the Drawings

FIG. 1 is a perspective view illustrating a pulse wave measuring device according to a first embodiment.
FIG. 2 is a perspective view from a surface side illustrating the pulse wave measuring device according to the first embodiment.
FIG. 3 is a perspective view from a back side illustrating the pulse wave measuring device according to the first embodiment.
FIG. 4 is a side view illustrating the pulse wave measuring device according to the first embodiment.
FIG. 5 is a cross-sectional view of a sensor portion.
FIG. 6 is an exploded perspective view of the sensor portion.
FIG. 7 is an exploded perspective view of a sensor unit.
FIG. 8 is a perspective view illustrating a pulse wave sensor according to the first embodiment.
FIG. 9 is a plan view illustrating the pulse wave sensor according to the first embodiment.
FIG. 10 is a cross-sectional view illustrating the pulse wave sensor according to the first embodiment.
FIG. 11 is a plan view illustrating a strain gauge according to the first embodiment.
FIG. 12 is a cross-sectional view (part 1) illustrating the strain gauge according to the first embodiment.
FIG. 13 is a cross-sectional view (part 2) illustrating the strain gauge according to the first embodiment.

### Description of Embodiments

Hereinafter, the embodiment for carrying out the invention will be described with reference to the drawings. In each figure, the same components are denoted by the same reference numerals, and duplicate descriptions may be omitted.

### <First Embodiment>

### [Pulse wave measuring device 1]

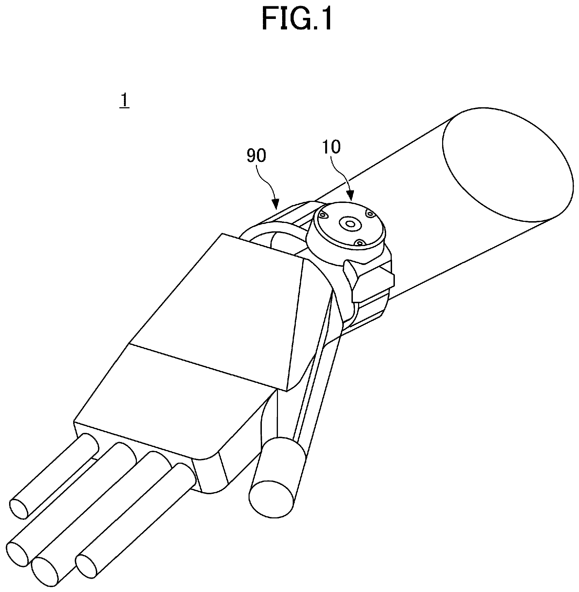

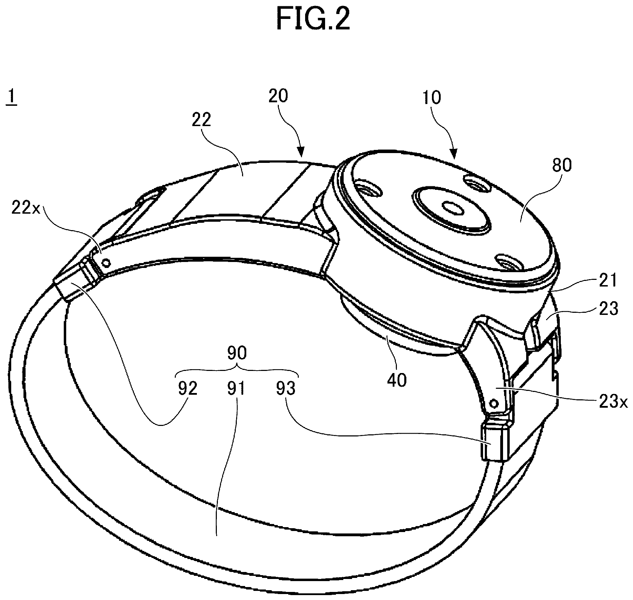

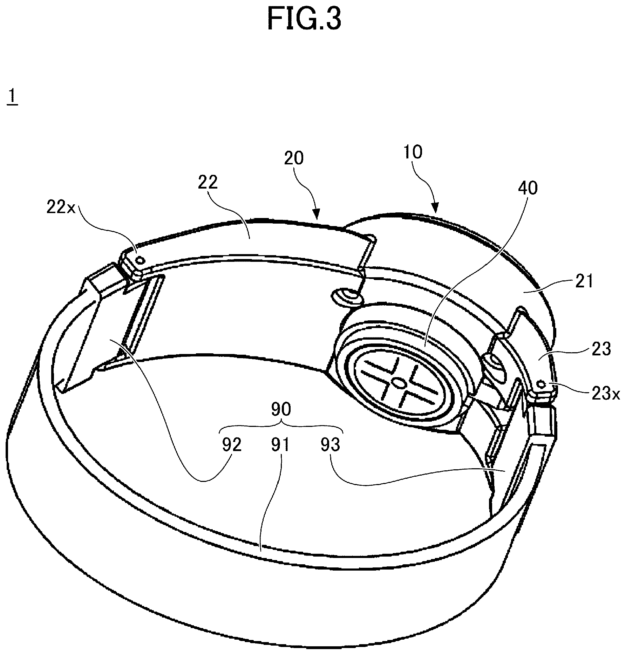

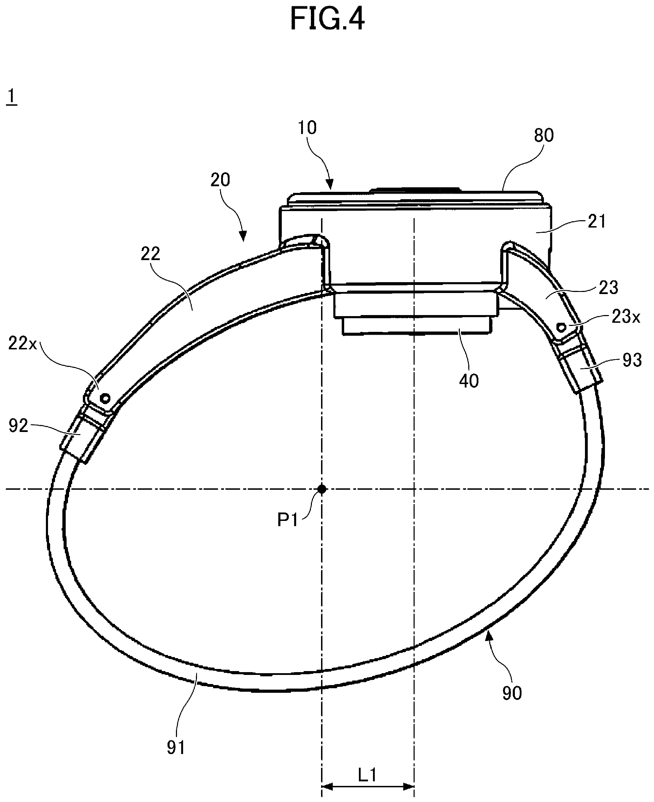

FIG. 1 is a perspective view illustrating the pulse wave measuring device according to the first embodiment, and shows a state in which the pulse wave measuring device is attached to a subject's wrist. FIG. 2 is a perspective view from the surface side illustrating the pulse wave measuring device according to the first embodiment. FIG. 3 is a perspective view from the back side illustrating a pulse wave measuring device according to the first embodiment. FIG. 4 is a side view illustrating the pulse wave measuring device according to the first embodiment.

As shown in FIG. 1, the pulse wave measuring device 1 is a wristwatch-type wearable device that can be attached to a subject's wrist. The pulse wave measuring device 1 mainly includes a sensor portion 10 and a band portion 90.

Referring to FIGS. 2 to 4, the sensor portion 10 includes at least a base 20 and a pulse wave sensor 40. The base 20 has a sensor placement member 21, a first curved member 22, and a second curved member 23. For example, a lid member 80 is provided on the sensor placement member 21. The detailed configuration of the sensor portion 10 will be described later.

Note that the pulse wave measuring device 1 may have any other configuration, so long as it has a sensor placement member 21, a first curved member 22, a second curved member 23, a pulse wave sensor 40, and a band portion 90.

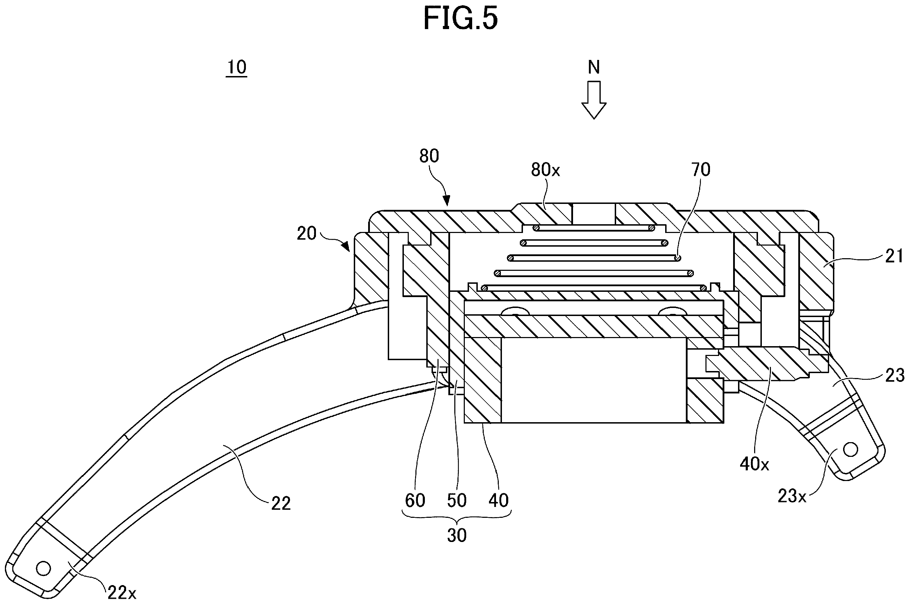

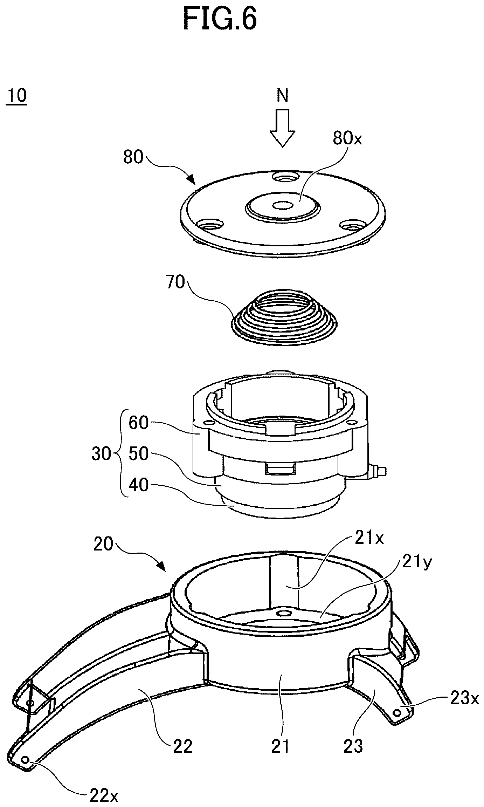

At the base 20, the sensor placement member 21 is a portion where the pulse wave sensor 40 is arranged. One end of the first curved member 22 is connected to the sensor placement member 21. In addition, one end of the second curved member 23 is connected to the sensor placement member 21. In a plan view, the first curved member 22 and the second curved member 23 are connected to opposite sides across the sensor placement member 21. Note that the plan view refers to viewing an object in a direction normal to an upper surface of the lid member 80 (N direction shown in FIGS. 5 and 6 described later).

The band portion 90 is a band-like portion for attaching the sensor portion 10 to the subject's wrist, and is configured so as to be wound around the subject's wrist from the outside. The band portion 90 is connected to another end of the first curved member 22, and another end of the second curved member 23.

The band portion 90 includes, for example, a band member 91, a first connecting member 92, and a second connecting member 93. The band member 91 is formed of, for example, resin, rubber, cloth, or the like, and has elasticity. The first connecting member 92 and the second connecting member 93 are members for connecting the band member 91 to the first curved member 22 and the second curved member 23. The first connecting member 92 and the second connecting member 93 can be formed of resin, rubber, or the like, for example.

In the illustrated example, one end of the band member 91 is inserted into a groove provided at one end of the first connecting member 92, and fixed thereto. Another end of the first connecting member 92 is attached, in a manner of being swingable about a single axis, to the attachment part 22x provided at the first curved member 22. In addition, another end of the band member 91 is inserted into a groove provided at one end of the second connecting member 93, and fixed thereto. Another end of the second connecting member 93 is attached, in a manner of being swingable about a single axis, to the attachment part 23x provided at the second curved member 23.

The first connecting member 92 and the second connecting member 93 can be provided as necessary. That is, the band portion 90 may consist of only the band member 91, one end of the band member 91 may be directly attached to the attachment part 22x of the first curved member 22 in a swingable manner, and another end of the band member 91 may be directly attached to the attachment part 23x of the second curved member 23 in a swingable manner.

The band member 91 may be a continuous single structure as that in the illustrated example, or may consist of a plurality of structures. For example, a first band-like body having one end fixed to the first connecting member 92, and a second band-like body having one end fixed to the second connecting member 93 may be provided, and another end of the first band-like body and another end of the second band-like body may be detachably connected by a hook-and-loop fastener or the like.

The first curved member 22 and the second curved member 23 are curved so that their respective centers approximately are directed toward the center P1 of the wrist (see FIG. 4) when the pulse wave measuring device 1 is attached to the subject's wrist. The curvatures of the first curved member 22 and the second curved member 23 are different. The radius of curvature of the first curved member 22 is larger than that of the second curved member 23. In other words, the curvature of the first curved member 22 is smaller than that of the second curved member 23. The preferred curvatures of the first curved member 22 and the second curved member 23 may differ between the pulse wave measuring device 1 for men and the pulse wave measuring device 1 for women. For example, the curvature of the first curved member 22 is preferably about 70 mm to 90 mm, and the curvature of the second curved member 23 is preferably about 20 mm to 30 mm.

Further, the lengths of the first curved member 22 and the second curved member 23 are different. The first curved member 22 is longer than the second curved member 23. The lengths of the first curved member 22 and the second curved member 23 are compared based on the lengths of portions closest to the center P1 of the first curved member 22 and the second curved member 23 in the side view shown in FIG. 4. For example, the length of the first curved member 22 is preferably about 70 mm to 90 mm, and the length of the second curved member 23 is preferably about 8 mm to 10 mm. However, the length, obtained by adding the length of the first curved member 22 and the length of the second curved member 23, is preferably equal to or less than half of the wrist circumference.

As shown in FIG. 4, in the pulse wave measuring device 1, the center of the pulse wave sensor 40 (the center of the strain body 42 described later) is offset by L1 from the center P1 of the subject's wrist. Although the position of the center P1 of the subject's wrist varies from person to person, in the pulse wave measuring device 1, L1 is designed to be about 15 mm to 20 mm. Note that L1 is an offset in the direction orthogonal to the normal to the upper surface of the lid member 80 (N direction shown in FIGS. 5 and 6 described later) from the center P1.

The pulse wave measuring device 1 is attached to the subject's wrist using the band portion 90 so that, for example, the pulse wave sensor 40 is positioned near the subject's radial artery. The pulse wave captures a volume change of the blood vessels generated as the heart pumps blood as a waveform, and the pulse wave measuring device 1 can monitor the volume change of the blood vessels.

Since the curvatures of the first curved member 22 and the second curved member 23 of the pulse wave measuring device 1 are different, it is easy to attach the pulse wave measuring device 1 along the subject's wrist even when the center of the pulse wave sensor 40 is offset from the center of the subject's wrist. Thus, the pulse wave sensor 40 can be stably positioned near the subject's radial artery, so that the pulse wave of the subject can be stably measured.

In addition, when the lengths of the first curved member 22 and the second curved member 23 are different, it is easier to attach the pulse wave measuring device 1 along the subject's wrist. As a result, the pulse wave sensor 40 can be more stably positioned near the subject's radial artery, and thus the pulse wave of the subject can be more stably measured.

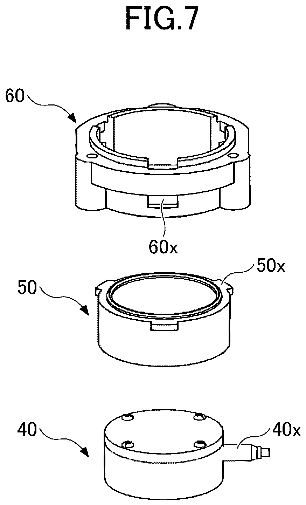

FIG. 5 is a cross-sectional view of the sensor portion. FIG. 6 is an exploded perspective view of the sensor portion. FIG. 7 is an exploded perspective view of the sensor unit. As shown in FIGS. 5 to 7, the sensor portion 10 includes, for example, a base 20, a sensor unit 30, a biasing member 70, and a lid member 80. Further, the sensor unit 30 includes, for example, a pulse wave sensor 40, a housing member 50, and a holding member 60.

In this embodiment, for convenience, the side of the sensor portion 10, where the lid member 80 is provided, is referred to as an "upper side", and the side of the sensor portion 10, where the base 20 is provided, is referred to as a "lower side". In addition, the surface located on the upper side of each portion is referred to as an "upper surface", and the surface located on the lower side of each portion is referred to as a "lower surface". However, the sensor portion 10 can also be used in an inverted state. The sensor portion 10 can also be arranged at any angle. In addition, the plan view refers to viewing the object from the upper side to the lower side along the direction normal to the upper surface of the lid member 80 (N direction shown in FIGS. 5 and 6). The plane shape refers to the shape of the object when viewing the object in the direction of the normal.

Regarding the base 20, the sensor placement member 21 is a portion where the sensor unit 30 is housed. As described later, since the biasing member 70 is housed in the holding member 60 constituting the sensor unit 30, the sensor placement member 21 may be considered to accommodate the sensor unit 30 and the biasing member 70.

The sensor portion 10 has, for example, a lid member 80 on a side of the sensor placement member 21 opposite to the direction in which the strain body 42 described later is exposed. For example, the lid member 80 is attached, from above the biasing member 70, to the sensor placement member 21 to accommodate the sensor unit 30 and the biasing member 70. The lid member 80 may be attached to the sensor placement member 21 by an appropriate means such as a screw, for example.

The lid member 80 may be provided with a marker 80x. Preferably, the center of the marker 80x is aligned with the center of the strain body 42 described later in a plan view. For example, the marker 80x may be provided at a position overlapping a load part 42c of the strain body 42 described later in a plan view and not overlapping a base part 42a thereof in a plan view. The marker 80x is, for example, a projection projecting from the upper surface of the lid member 80. A non-through groove or a through hole may be provided at the center of the projection. By providing, on the upper surface of the lid member 80, the marker 80x having a center which is aligned with the center of the strain body 42 described later in a plan view, the marker 80x can be used as a marker when arranging the pulse wave measuring device 1 near the subject's radial artery.

A groove 21x for positioning the sensor unit 30 to the base 20 is provided on the inner surface of the sensor placement member 21. In the illustrated example, three grooves 21x having a longitudinal direction along the thickness direction of the sensor placement member 21 are provided at approximately equal intervals in the circumferential direction of the sensor placement member 21. However, if the sensor unit 30 can be positioned at the base 20, the shape and number of the grooves 21x are not limited to the illustrated example. On the lower end side of the inner surface of the sensor placement member 21, a substantially ring-shaped receiving part 21y is provided so as to project from the inner surface to the center side in a plan view. The receiving part 21y serves as a stopper for the sensor unit 30. The center side of the receiving part 21y is open.

As shown in FIG. 7, the pulse wave sensor 40 is a substantially cylindrical member, and is formed so as to be housed in the housing member 50. The upper surface side and the lower surface side of the pulse wave sensor 40 are closed. A connecting part 40x for connecting a cable or the like may be provided on the outer surface of the pulse wave sensor 40. For example, the pulse wave sensor 40 can transmit and receive electrical signals to and from an external circuit or the like by wire via a cable or the like connected to the connecting part 40x. The pulse wave sensor 40 may be configured to transmit and receive electrical signals to and from an external circuit or the like wirelessly without providing the connecting part 40x. The detailed configuration of the pulse wave sensor 40 will be described later.

The housing member 50 houses the pulse wave sensor 40 therein. The housing member 50 is a substantially cylindrical member, and its upper surface side is closed and its lower surface side is open. On the upper end side of the outer surface of the housing member 50, a plurality of projection parts 50x projecting outward from the outer surface of the housing member 50 are provided to engage with the holding member 60. In the illustrated example, three projection parts 50x are provided at approximately equal intervals in the circumferential direction of the housing member 50. However, if the housing member 50 can engage with the holding member 60, the shape and number of the projection parts 50x are not limited to the illustrated example. In addition, although not shown, an opening for projecting the connecting portions 40x of the pulse wave sensor 40 outward is provided on the outer surface of the housing member 50. The pulse wave sensor 40 is housed in the housing member 50 from the lower surface side of the housing member 50, while the lower surface side of the pulse wave sensor 40 projects from the housing member 50.

The holding member 60 holds the pulse wave sensor 40 housed in the housing member 50. The holding member 60 is a substantially cylindrical member, and its upper surface side and lower surface side are open. On an outer surface of the holding member 60, an opening 60x for engaging with the projection part 50x of the housing member 50 is provided at a position where it can engage with the projection part 50x. When the projection part 50x of the housing member 50 engages with the opening 60x of the holding member 60, the pulse wave sensor 40 housed in the housing member 50 is held by the holding member 60.

In the state of being held by the holding member 60, a part of the pulse wave sensor 40 and a part of the housing member 50 project to the lower side of the holding member 60. In addition, in the state of being held by the holding member 60, the upper surface of the housing member 50 does not reach the uppermost part of the holding member 60. That is, a recess is formed by the upper surface of the housing member 50 and a part of the inner surface of the holding member 60. The biasing member 70 is housed in the recess.

As shown in FIG. 5, when the lid member 80 is attached to the sensor placement member 21 housing the sensor unit 30 and the biasing member 70 from above the biasing member 70, the biasing member 70 can bias the pulse wave sensor 40 toward the N direction. Note that the holding member 60 of the sensor unit 30 is fixed to the sensor placement member 21. Therefore, the pulse wave sensor 40 and the housing member 50 move integrally by the act of the biasing member 70. In other words, the pulse wave sensor 40 and the housing member 50 are held by the sensor placement member 21 in a state of being capable of moving in the axial direction (N direction in FIG. 5) of the sensor placement member 21.

That is, when the pulse wave measuring device 1 is attached to the subject, the biasing member 70 can bias the pulse wave sensor 40 toward the subject. The biasing member 70 is, for example, a coil spring, but may be a leaf spring or the like. The biasing member 70 may be formed of, for example, metal, resin, rubber or the like. Note that the biasing member 70 may be an air pump using an electric motor, or the like.

When the biasing member 70 is a coil spring, the biasing member 70 is preferably a conical coil spring. When the biasing member 70 is a conical coil spring, the height of the sensor placement member 21 when compressed can be reduced compared with the case of a cylindrical coil spring, thereby the low-profile configuration of the sensor placement member 21 can be achieved. When the biasing member 70 is a conical coil spring, it is preferable to arrange the portion of the conical coil spring having a small coil diameter toward the lid member 80, so that the biasing member 70 can be stably arranged.

In this manner, the sensor portion 10 of the pulse wave measuring device 1 has a biasing mechanism in which the pulse wave sensor 40 is biased toward the subject by the biasing member 70, and thus an appropriate initial pressure can be applied to the subject's radial artery. As a result, in the pulse wave measuring device 1, a good fit between the subject and the pulse wave sensor 40 can be obtained, and the measurement accuracy of the pulse wave can be improved.

### [Pulse wave sensor 40]

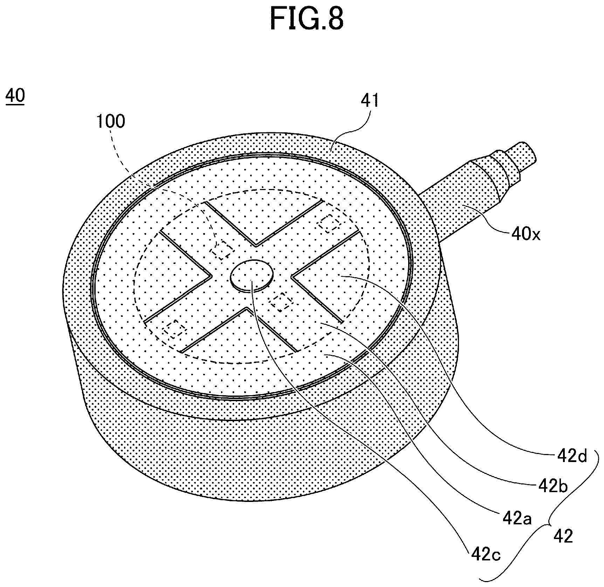

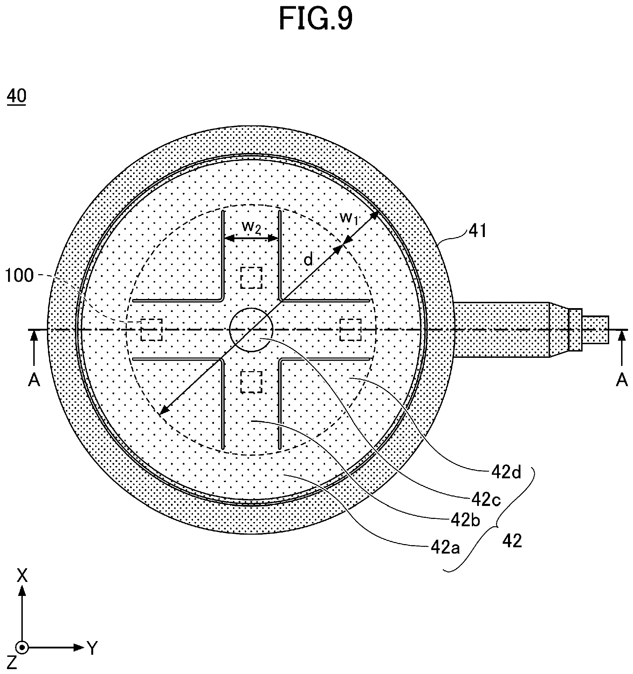

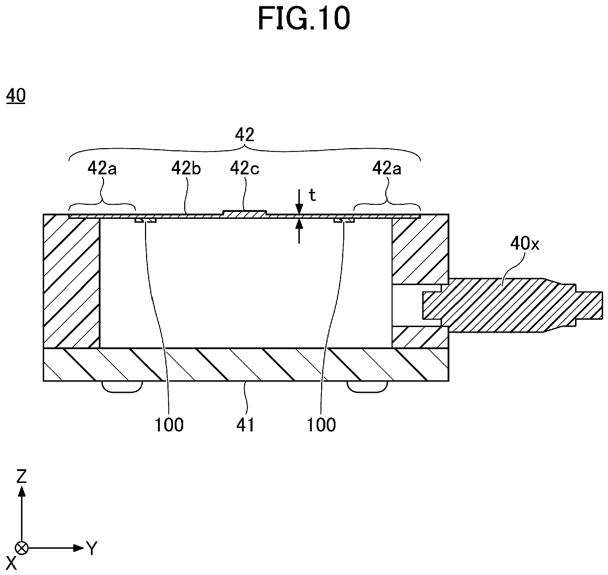

FIG. 8 is a perspective view illustrating the pulse wave sensor according to the first embodiment. FIG. 9 is a plan view illustrating the pulse wave sensor according to the first embodiment. FIG. 10 is a cross-sectional view illustrating the pulse wave sensor according to the first embodiment, showing a cross-section taken along line A-A in FIG. 9. Note that FIGS. 8 to 10 have different viewing directions from FIGS. 5 to 7, and the lower surface of the pulse wave sensor 40 in FIGS. 5 to 7 is the upper surface in FIGS. 8 to 10.

Referring to FIGS. 8 to 10, the pulse wave sensor 40 has a case 41, a strain body 42, and a strain gauge 100. Referring to FIGS. 4 or the like in addition to FIGS. 8 to 10, the pulse wave sensor 40 is held in a holding member 60 in a state that the strain body 42 is exposed from the sensor placement member 21 of the base 20 and thus in contact with the subject.

The pulse wave sensor 40 preferably projects from the sensor placement member 21 of the base 20 toward the subject. In the case of the pulse wave sensor 40 projecting from the sensor placement member 21 toward the subject, the projection amount is preferably about 3 mm to 7 mm. In this case, for example, if the spring constant of the biasing member 70 is 0.33 N/mm, a load of about 100 g can be applied to the subject. As a result, a good fit between the subject and the pulse wave sensor 40 can be obtained, and the measurement accuracy of the pulse wave can be improved.

In this manner, the pulse wave sensor 40 includes the strain body 42 with the strain gauge 100 described later arranged therein. Then, at least a portion of the pulse wave sensor 40 is situated in the inside of the sensor placement member 21 and is held so as to be capable of moving in the axial direction of the sensor placement member 21, and the strain body 42 is exposed from the sensor placement member 21 to be capable of contacting the subject. In addition, since the pulse wave sensor 40 projects beyond the sensor placement member 21 toward the subject, an appropriate initial pressure can be applied to the subject's radial artery.

The strain body 42 has a base part 42a, an arm part 42b, a load part 42c, and extending parts 42d. The strain body 42 is of, for example, four-fold symmetric shape in a plan view. As the material of the strain body 42, for example, SUS (stainless steel), copper, aluminum, and the like can be used. The strain body 42 is, for example, in the form of a flat plate, and each component is integrally formed by, for example, a pressing process or the like. The strain body 42 may be flat, or may be in the form of a dome or the like so that a portion close to the subject is convex. The thickness t of the strain body 42 excluding the load part 42c is, for example, constant. The thickness t is, for example, 0.01 mm to 0.25 mm.

Note that in the description of the pulse wave sensor 40 in FIGS. 8 to 10, for convenience, the side of the strain body 42, where the load part 42c is provided, is referred to as an "upper side" or "one side", and the side of the strain body 42, where the load part 42c is not provided, is referred to as a "lower side" or "the other side". In addition, the surface on the side of each part where the load part 42c is provided is referred to as an "upper surface" or "one surface", and the surface on the side of each part where the load part 42c is not provided is referred to as a "lower surface" or "the other surface". However, the pulse wave sensor 40 can be used in an inverted state or arranged at any angle. In addition, the plan view refers to viewing the object in the direction normal to the upper surface of the strain body 42, and the plane shape refers to the shape of the object when viewing the object in the direction normal to the upper surface of the strain body 42.

Regarding the pulse wave sensor 40, the case 41 holds the strain generating body 42. The case 41 is cylindrical, and its lower surface side is closed and its upper surface side is opened. The case 41 can be formed of, for example, metal, resin or the like. In order to close an opening on the upper surface side of the case 41, a substantially disk-shaped strain body 42 is fixed by an adhesive or the like. The strain body 42 is provided with the strain gauge 100 as a part for detecting the pulse wave.

Regarding the strain body 42, the base part 42a is a circular frame-like (ring-like) region outside the circular dashed line shown in FIGS. 8 and 9. Note that the region inside the circular dashed line may be called a circular opening. That is, the base part 42a of the strain body 42 has a circular opening. The width w₁ of the base part 42a is, for example, 1 mm or more and 5 mm or less. The inner diameter d (i.e., the diameter of the circular opening) of the base part 42a is, for example, 5 mm or more and 40 mm or less.

The arm part 42b is provided so as to bridge the inner side of the base part 42a. The arm part 42b has, for example, two arms intersecting in a cross shape in a plan view, and a region where the two arms intersect includes a center of the circular opening. In the example of FIG. 9, one arm constituting the cross has the X direction as its longitudinal direction, and the other arm constituting the cross has the Y direction as its longitudinal direction, and the two arms are orthogonal to each other. Preferably, each of the two arms intersecting orthogonally is located inside the inner diameter d (diameter of the circular opening) of the base 42a, and is as long as possible. That is, the length of each arm is preferably approximately equal to the diameter of the circular opening. In each arm constituting the arm part 42b, the width w₂ of the region other than the intersecting region is constant, for example, 1 mm or more and 5 mm or less. Although it is not essential that the width w₂ be constant, it is preferable so that the strain can be detected linearly by making the width w2 constant.

The load part 42c is provided on the arm part 42b. The load part 42c is provided, for example, in an area where two arms constituting the arm part 42b intersect. The load part 42c projects from the upper surface of the arm part 42b. The projection amount of the load part 42c relative to the upper surface of the arm part 42b is, for example, about 0.1 mm. The arm part 42b has flexibility, and elastically deforms when a load is applied to the load part 42c.

The four extending parts 42d are sectorial portions extending from the inner side of the base part 42a toward the arm part 42b in a plan view. A gap of about 1 mm is provided between each extending part 42d and the arm part 42b. Note that if the gap is about 0.05 mm to 0.2 mm, for example, it is possible to prevent contamination from entering the inside of the case 41 from the outside. Since the extending parts 42d do not contribute to the sensing of the pulse wave sensor 40, they may not be provided. The pulse wave sensor 40 includes a shield cable, a flexible substrate, or the like (not shown) for inputting and outputting electrical signals to and from the outside.

The strain gauge 100 is provided on the strain body 42. The strain gauge 100 can be provided on the lower surface side of the arm part 42b, for example. Since the arm part 42b has a flat plate shape, the strain gauge can be attached easily. One or more strain gauges 100 may be provided, and in this embodiment, four strain gauges 100 are provided. By providing four strain gauges 100, strain can be detected by a full bridge.

Two of the four strain gauges 100 are arranged to be close to the load part 42c (i.e., the center of the circular opening) on the arm having the X direction as its longitudinal direction, so as to face each other with the load part 42c interposed therebetween in a plan view. The other two of the four strain gauges 100 are arranged to be close to the base part 42a on the arm having the Y direction as its longitudinal direction, so as to face each other with the load part 42c interposed therebetween in a plan view. With such an arrangement, compressive and tensile forces can be effectively detected, and a large output can be obtained by the full bridge.

The pulse wave sensor 40 is fixed, when in use, to the subject's arm so that the load part 42c contacts the subject's radial artery. When a load is applied to the load part 42c in response to the subject's pulse wave and thus the arm part 42b is elastically deformed, the resistance value of the resistor of the strain gauge 100 changes. The pulse wave sensor 40 can detect the pulse wave based on the change in the resistance value of the resistor of the strain gauge 100 caused by the deformation of the arm part 42b. The pulse wave is output as a periodic voltage change from, for example, a measuring circuit connected to the electrode of the strain gauge 100.

### [Strain gauge 100]

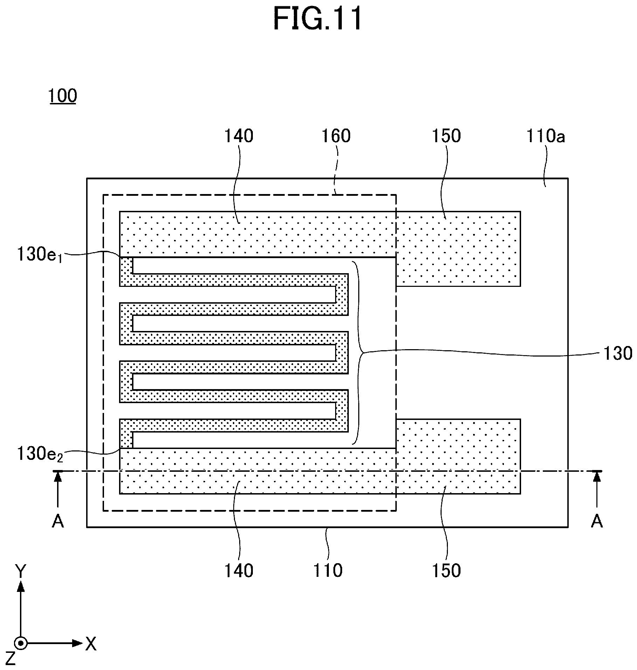

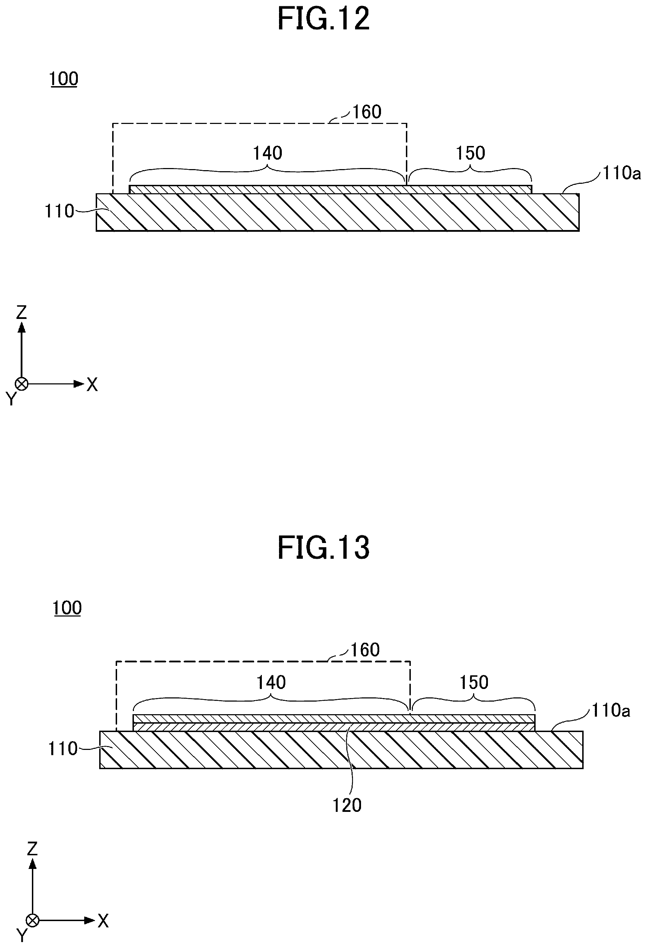

FIG. 11 is a plan view illustrating the strain gauge according to the first embodiment. FIG. 12 is a cross-sectional view illustrating the strain gauge according to the first embodiment, showing a cross-section taken along line A-A in FIG. 11. Referring to FIGS. 11 and 12, the strain gauge 100 includes a base material 110, a resistor 130, a wiring 140, an electrode 150, and a cover layer 160. Note that in FIG. 11, for convenience, only the outer edge of the cover layer 160 is shown as a dashed line. Note that the cover layer 160 may be provided as necessary.

Note that in the description of the strain gauge 100 in FIGS. 11 and 12, for convenience, the side of the strain gauge 100, where the resistor 130 of the base material 110 is provided, is referred to as the "upper side", and the side of the strain gauge 100, where the resistor 130 is not provided, is referred to as the "lower side". In addition, the surface located on the upper side of each portion is referred to as the "upper surface", and the surface located on the lower side of each portion is referred to as the "lower surface". However, the strain gauge 100 can also be used in an inverted state. The strain gauge 100 can also be arranged at any angle. For example, in FIG. 10, the strain gauge 100 is attached to the arm part 42b in a state where the upper and lower portions are inverted from those of FIG. 12. That is, the base material 110 in FIG. 12 is attached to the lower surface of the arm part 42b with an adhesive or the like. In addition, the plan view refers to viewing the object from the upper side to the lower side along the direction normal to the upper surface 110a of the base material 110. Then, the plane shape refers to the shape of the object when viewing the object in the direction of the normal.

The base material 110 serves as a base layer for forming the resistor 130 and the like. The base material 110 has flexibility. The thickness of the base material 110 is not particularly limited, and may be appropriately determined in accordance with the intended use or the like of the strain gauge 100. For example, the thickness of the base material 110 may be about 5 µm to 500 µm. Note that the thickness of the base material 110 is preferably in the range of 5 µm to 200 µm from the viewpoints of the transmissibility of the strain from the outer surface of the strain body 42 to the sensing part, and of the dimensional stability against environmental changes. In addition, from the viewpoint of the insulating property, the thickness of the base material 110 is preferably 10 µm or more.

The base material 110 is formed of an insulating resin film such as, for example, a PI (polyimide) resin, an epoxy resin, a PEEK (polyether ether ketone) resin, a PEN (polyethylene naphthalate) resin, a PET (polyethylene terephthalate) resin, a PPS (polyphenylene sulfide) resin, an LCP (liquid crystal polymer) resin, and a polyolefin resin. Note that the film refers to a member having a thickness of about 500 µm or less and having flexibility.

When the base material 110 is formed of an insulating resin film, the insulating resin film may contain fillers, impurities, etc. For example, the base material 110 may be formed of an insulating resin film containing fillers such as silica and alumina.

Materials other than resin for the base material 110 include, for example, crystalline materials such as SiO₂, ZrO₂ (including YSZ), Si, Si₂N₃, Al₂O₃ (including sapphire), ZnO, and perovskite-based ceramics (CaTiO₃, BaTiO₃). In addition to the afore-mentioned crystalline materials, amorphous glass or the like may be used as the base material 110. Further, a metal such as aluminum, an aluminum alloy (duralumin), titanium or the like may be used as the material of the base material 110. When a metal is used, an insulating film is provided on the metal base material 110.

The resistor 130 is a thin film formed in a predetermined pattern on the upper side of the base material 110. Regarding the strain gauge 100, the resistor 130 is a sensing part that receives strain and then causes a resistance change. The resistor 130 may be formed directly on the upper surface 110a of the base material 110, or may be formed above the upper surface 110a of the base material 110 via another layer. Note that in FIG. 11, the resistor 130 is shown in a dark pear pattern for convenience.

The resistor 130 is of a structure in which a plurality of elongated portions are arranged at a predetermined interval, having the longitudinal directions thereof oriented in the same direction (X direction in the example shown in FIG. 11), and the end portions of adjacent elongated portions are connected to each other alternately and thus have a zigzag pattern as a whole. The longitudinal direction of the plurality of elongated portions is a grid direction, and the direction perpendicular to the grid direction is a grid width direction (Y direction in the example shown in FIG. 11).

Regarding the resistor 130, the X- side end of the elongated portion located at the most Y+ side bends in the Y+ direction, and reaches one end part 130e₁ in the grid width direction of the resistor 130. In addition, the X- side end of the elongated portion located at the most Y-side bends in the Y-direction, and reaches the other end part 130e₂ in the grid direction of the resistor 130. Each of the end parts 130ei, 130e₂ is electrically connected to the electrode 150 via the wiring 140. In other words, the wiring 140 electrically connects each of the end parts 130e₁, 130e₂ to the corresponding electrode 150 in the grid width direction of the resistor 130.

The resistor 130 may be formed, for example, of a material containing Cr (chromium), a material containing Ni (nickel), or a material containing both Cr and Ni. That is, the resistor 130 may be formed of a material containing at least one of Cr and Ni. The material containing Cr includes, for example, a Cr mixed phase film. The material containing Ni includes, for example, Cu-Ni (copper nickel). The material containing both Cr and Ni include, for example, Ni-Cr (nickel-chromium).

Here, the Cr mixed phase film is a film in which Cr, CrN, Cr₂N, and the like are mixed. The Cr mixed phase film may contain unavoidable impurities such as chromium oxide.

The thickness of the resistor 130 is not particularly limited, and may be appropriately determined in accordance with the intended use or the like of the strain gauge 100. For example, the thickness of the resistor 130 may be about 0.05 µm to 2 µm. Especially when the thickness of the resistor 130 is 0.1 µm or more, the crystallinity (For example, the crystallinity of α-Cr) of the crystals constituting the resistor 130 is improved. When the thickness of the resistor 130 is 1 µm or less, (i) cracks in the film and (ii) a warpage of the film from the base material 110 caused by internal stresses in the film constituting the resistor 130 are reduced.

In view of the difficulty in generating lateral sensitivity and the wire breakage countermeasures, it is preferable that the width of the resistor 130 is 10 µm or more and 100 µm or less. Furthermore, the width of the resistor 130 is preferably 10 µm or more and 70 µm or less, and more preferably 10 µm or more and 50 µm or less.

For example, when the resistor 130 is a Cr mixed phase film, the stability of the gauge characteristics can be improved by using α-Cr (alpha chromium), which is a stable crystalline phase, as a main component. For example, when the resistor 130 is a Cr mixed phase film, by using α-Cr as the main component in the resistor 130, the gauge rate of the strain gauge 100 can be 10 or more, and the gauge rate temperature coefficient TCS and the resistance temperature coefficient TCR can be set within the range of -1000 ppm/°C to +1000 ppm/°C. Here, the term "main component" refers to a component comprising 50 wt% or more of the total material constituting the resistor. From the viewpoint of improving the gauge characteristics, the resistor 130 preferably contains 80 wt% or more of α-Cr. Furthermore, from the same viewpoint, the resistor 130 preferably contains 90 wt% or more of α-Cr. Note that α-Cr is Cr having a bcc structure (body-centered cubic lattice structure).

When the resistor 130 is a Cr mixed phase film, CrN and Cr₂N contained in the Cr mixed phase film are preferably 20 wt% or less. When CrN and Cr₂N contained in the Cr mixed phase film are 20 wt% or less, the decrease in the gauge ratio of the strain gauge 100 can be suppressed.

In addition, it is preferable that the ratio of CrN and Cr₂N in the Cr mixed phase film is such that the proportion of Cr₂N is 80 wt% or more and less than 90 wt% relative to the sum of the weights of CrN and Cr₂N. More preferably, the ratio is such that the proportion of Cr₂N is 90 wt% or more and less than 95 wt% relative to the sum of the weights of CrN and Cr₂N. Cr₂N has semiconducting properties. Therefore, the decrease in TCR (negative TCR) becomes more pronounced when the proportion of Cr₂N mentioned above is set to 90 wt% or more and less than 95 wt%. Furthermore, the ceramization of the resistor 130 can be reduced by setting the proportion of Cr₂N mentioned above to 90 wt% or more and less than 95 wt%. Therefore, brittle fracture of the resistor 130 can be reduced.

On the other hand, CrN has the advantage of being chemically stable. By including more CrN in the Cr mixed phase film, the possibility of the generation of an unstable N can be reduced, and thus a stable strain gauge can be obtained. Here, "unstable N" means a small amount of N₂ or atomic N that can be present in the film of the Cr mixed phase film. These unstable N may escape from the film depending on the external environment (e.g., high temperature environment). As the unstable N escapes from the film, the film stress of the Cr mixed phase film may change.

The wiring 140 is provided on the base material 110. The wiring 140 is electrically connected to the resistor 130 and the electrode 150. The wiring 140 is not limited to a straight line, and can be of any pattern. The wiring 140 also can be of any width and any length. Note that in FIG. 11, for convenience, the wiring 140 is shown in a pear pattern thinner than the resistor 130.

The electrode 150 is provided on the base material 110. The electrode 150 is electrically connected to the resistor 130 via the wiring 140. The electrode 150 is formed in a substantially rectangular shape with a wider width than the wiring 140 in a plan view. The electrode 150 includes a pair of electrodes for outputting to the outside a change in the resistance value of the resistor 130 caused by strain. A lead wire or the like for external connection is joined to the electrode 150. A metal layer having low resistance, such as copper, or a metal layer having good solderability, such as gold, may be laminated on the upper surface of the electrode 150. Although the resistor 130, the wiring 140, and the electrode 150 are assigned to different reference numerals for convenience, they can be integrally formed of the same material in the same process. Note that in FIG. 11, the electrode 150 is shown in the same pear pattern as the wiring 140 for convenience.

The cover layer 160 is provided on the base material 110 as necessary. The cover layer 160 is provided on the upper surface 110a of the base material 110 so as to cover the resistor 130 and the wiring 140 and expose the electrode 150. The material of the cover layer 160 may be, for example, an insulating resin such as a PI resin, an epoxy resin, a PEEK resin, a PEN resin, a PET resin, a PPS resin, or a composite resin (for example, silicone resin, polyolefin resin). Note that the cover layer 160 may contain a filler or a pigment. The thickness of the cover layer 160 is not particularly limited, and can be appropriately selected according to the purpose. For example, the thickness of the cover layer 160 can be about 2 µm to 30 µm. By providing the cover layer 160, it is possible to suppress the occurrence of mechanical damage or the like in the resistor 130. Moreover, by providing the cover layer 160, it is possible to protect the resistor 130 from moisture or the like.

Regarding the strain gauge 100, when a Cr mixed phase film is used as the material of the resistor 130, high sensitivity and miniaturization can be realized. For example, an output of the conventional strain gauge is about 0.04 mV/2 V, and in contrast, an output of 0.3 mV/2 V or more can be obtained when the Cr mixed phase film is used as the material of the resistor 130. In addition, the size of the conventional strain gauge (gauge length × gauge width) is about 3 mm×3 mm, and in contrast, the size (gauge length × gauge width) when the Cr mixed phase film is used as the material of the resistor 130 can be reduced to be about 0.3 mm×0.3 mm.

Therefore, the strain gauge 100 using the Cr mixed phase film as the material of the resistor 130 is particularly suitable for the pulse wave measuring device 1, in which the arrangement of the strain body 42 in a narrow position, and the detection of extremely minute fluctuations occurring in the radial artery are necessary. Moreover, the strain gauge 100 using the Cr mixed phase film as the material of the resistor 130 has higher resistance compared to the conventional strain gauge. Therefore, since the power consumption can be reduced when driven by batteries, the life of batteries can be extended.

### [Method of manufacturing strain gauge]

Hereinafter, a method of manufacturing the strain gauge 100 will be described. To manufacture the strain gauge 100, first, a base material 110 is prepared and a metal layer (referred to as a metal layer A for convenience) is formed on the upper surface 110a of the base material 110. The metal layer A is a layer that is finally patterned to be a resistor 130, a wiring 140, and an electrode 150. Therefore, the material and thickness of the metal layer A are the same as those of the resistor 130, the wiring 140, and the electrode 150.

The metal layer A can be formed by, for example, a magnetron sputtering method targeting a raw material capable of forming the metal layer A. The metal layer A may be formed by a reactive sputtering method, a vapor deposition method, an arc ion plating method, or a pulsed laser deposition method, instead of the magnetron sputtering method.

Note that the metal layer A may be formed after an underlayer is formed on the upper surface 110a of the base material 110. For example, a functional layer with a predetermined film thickness may be vacuum formed on the upper surface 110a of the base material 110 by conventional sputtering method. By providing the underlayer in this manner, the gauge characteristics of the strain gauge 100 can be stabilized.

In the present application, the functional layer refers to a layer having a function of promoting crystal growth of at least the upper metal layer A (resistor 130). The functional layer further preferably has a function of preventing the oxidation of the metal layer A by oxygen or moisture contained in the base material 110 and/or improving the adhesion between the base material 110 and the metal layer A. The functional layer may further have other functions.

The insulating resin film constituting the base material 110 may contain oxygen and moisture, and Cr may form a self-oxidizing film. Therefore, especially when the metal layer A contains Cr, it is preferable to form a functional layer having a function of preventing the oxidation of the metal layer A.

Thus, by providing the functional layer in the lower layer of the metal layer A, crystal growth of the metal layer A can be promoted, and the metal layer A composed of a stable crystal phase can be produced. As a result, the stability of the gauge characteristics is improved for the strain gauge 100. Further, the material constituting the functional layer diffuses into the metal layer A, thereby improving the gauge characteristics of the strain gauge 100.

FIG. 13 is a cross-sectional view (part 2) illustrating the strain gauge according to the first embodiment. FIG. 13 shows the cross-sectional shape of the strain gauge 100 when the functional layer 120 is provided as the underlayer of the resistor 130, the wiring 140, and the electrode 150.

The planar shape of the functional layer 120 may be patterned substantially the same as, for example, the planar shape of the resistor 130, the wiring 140, and the electrode 150. However, the planar shape of the functional layer 120, and the planar shape of the resistor 130, the wiring 140, and the electrode 150 may not be substantially the same. For example, when the functional layer 120 is formed of an insulating material, the functional layer 120 may be patterned in a shape different from the planar shape of the resistor 130, the wiring 140, and the electrode 150. In this case, the functional layer 120 may be formed in a flat manner in a region where the resistor 130, the wiring 140, and the electrode 150 are formed, for example. Alternatively, the functional layer 120 may be formed in a flat manner over the entire upper surface of the base material 110.

Next, the metal layer A is patterned by photolithography to form the resistor 130, the two wirings 140, and the two electrodes 150 in a planar shape shown in FIG. 11.

After the resistor 130, the wirings 140, and the electrodes 150 are formed, a cover layer 160 may be formed on the upper surface 110a of the base material 110. The cover layer 160 covers the resistor 130 and the wirings 140, but the electrodes 150 may be exposed from the cover layer 160. For example, the cover layer 160 may be formed by laminating a semi-cured thermosetting insulating resin film on the upper surface 110a of the base material 110 so as to cover the resistor 130 and the wirings 140 and expose the electrodes 150, and then heating the insulating resin film to cure it. By the above steps, the strain gauge 100 is completed.

Although the preferred embodiments and the like have been described in detail, they are not limited to the above-described embodiments and the like, and various modifications and substitutions can be made to the above-described embodiments and the like, without departing from the scope described in the claims.

This international application claims priority under Japanese Patent Application No. 2021-199963 filed on December 9, 2021, and the entire contents of Japanese Patent Application No. 2021-199963 are incorporated herein.

### Description of Symbols

1 pulse wave measuring device, 10 sensor portion, 20 base, 21 sensor placement member, 21x groove, 21y receiving part, 22 first curved member, 22x attachment part, 23 second curved member, 23x attachment part, 30 sensor unit, 40 pulse wave sensor, 40x connecting part, 41 case, 42 strain body, 42a base part, 42b arm part, 42c loading part, 42d extending part, 50 housing member, 50x projection part, 60 holding member, 60x opening, 70 biasing member, 80 lid member, 80x marker, 90 band portion, 91 band member, 92 first connecting part, 93 second connecting part, 100 strain gauge, 110 base material, 110a upper surface, 130 resistor, 130ei, 130e₂ end part, 140 wiring, 150 electrode, 160 cover layer.

## Claims

1. A pulse wave measuring device attachable to a subject to be measured, comprising:
a pulse wave sensor having a strain gauge;
a sensor placement member in which the pulse wave sensor is arranged;
a first curved member having one end connected to the sensor placement member;
a second curved member having one end connected to the sensor placement member; and
a band member connected to another end of the first curved member and another end of the second curved member,
wherein the first curved member and the second curved member are connected to opposite sides across the sensor placement member in a plan view, and
wherein a curvature of the first curved member is different from a curvature of the second curved member.

2. The pulse wave measuring device according to claim 1, wherein a length of the first curved member is different from a length of the second curved member.

3. The pulse wave measuring device according to claim 1 or 2, comprising:
a biasing member biasing the pulse wave sensor toward the subject.

4. The pulse wave measuring device according to claim 3, wherein the biasing member is a coil spring.

5. The pulse wave measuring device according to claim 4, wherein the biasing member is a conical coil spring.

6. The pulse wave measuring device according to any one of claims 1 to 5, wherein the band member has elasticity.

7. The pulse wave measuring device according to any one of claims 1 to 6, wherein the pulse wave sensor includes a strain body on which the strain gauge is disposed, and at least a portion of the pulse wave sensor is situated in an inside of the sensor placement member and is held so as to be capable of moving in an axial direction of the sensor placement member, and the strain body is exposed from the sensor placement member to be capable of contacting the subject.

8. The pulse wave measuring device according to claim 7, comprising:
a lid member disposed on a side of the sensor placement member opposite to a direction in which the strain body is exposed,
wherein the lid member is provided with a marker aligned with a center of the strain body in a plan view.

9. The pulse wave measuring device according to claim 7 or 8, wherein the strain body includes:
a base part having a circular opening;
an arm part bridging an inner side of the base part; and
a load part provided on the arm part,
wherein a pulse wave is detected based on a change in a resistance value of the strain gauge in response to a deformation of the strain body.

10. The pulse wave measuring device according to claim 9, wherein the arm part has two arms intersecting in a cross shape in a plan view,
wherein an intersecting region of the arms includes a center of the circular opening, and
wherein the load part is provided in the intersecting region of the arms.

11. The pulse wave measuring device according to claim 10, wherein the strain gauge is provided as four strain gauges,
wherein two of the four strain gauges are arranged to be close to the load part on one of the arms having a first direction as a longitudinal direction thereof, and the two of the four strain gauges are arranged so as to face each other with the load part interposed therebetween in a plan view, and
wherein other two of the four strain gauges are arranged to be close to the base part on one of the arms having a second direction orthogonal to the first direction as a longitudinal direction thereof, and the other two of the four strain gauges are arranged so as to face each other with the load part interposed therebetween in a plan view.

12. The pulse wave measuring device according to any one of claims 1 to 11, wherein the pulse wave sensor projects beyond the sensor placement member toward the subject.

13. The pulse wave measuring device according to any one of claims 1 to 12, wherein the strain gauge has a resistor formed of a Cr mixed phase film.
